# EUROPÄISCHE PATENTSCHRIFT

(11) **EP 4 175 566 B1**
(45) Veröffentlichungstag und Bekanntmachung des Hinweises auf die Patenterteilung: **03.09.2025**
(21) Anmeldenummer: 21742053.8
(22) Anmeldetag: 29.06.2021
(51) Int. Cl.: A61B 17/22, A61B 17/32, A61B 17/00

(54) **VERFAHREN ZUR BESTIMMUNG EINER OPTIMALEN FREQUENZ EINER OSZILLIERENDEN BEWEGUNG EINES DURCH EINE KRAFT BESCHLEUNIGTEN PROJEKTILS EINER INTRAKORPORALEN LITHOTRIPSIEVORRICHTUNG**
METHOD FOR DETERMINING AN OPTIMAL FREQUENCY OF AN OSCILLATING MOVEMENT OF A FORCE-ACCELERATED PROJECTILE OF AN INTRACORPOREAL LITHOTRIPSY APPARATUS
PROCÉDÉ DE DÉTERMINATION D'UNE FRÉQUENCE OPTIMALE D'UN MOUVEMENT OSCILLANT D'UN PROJECTILE ACCÉLÉRÉ PAR UNE FORCE D'UN APPAREIL DE LITHOTRIPSIE INTRACORPORELLE

(30) Priorität: 01.07.2020 DE 102020117364
(43) Veröffentlichungstag der Anmeldung: 10.05.2023
(73) Patentinhaber: Karl Storz SE & Co. KG, 78532 Tuttlingen (DE)
(72) Erfinder: HINDING, Thomas, 78532 Tuttlingen (DE); GLÖGGLER, Bernhard, 78532 Tuttlingen (DE)
(86) Internationale Anmeldenummer: PCT/EP2021/067828
(87) Internationale Veröffentlichungsnummer: WO 2022/002926

(56) Entgegenhaltungen:
- EP-A1- 1 163 882
- EP-A1- 1 163 883
- DE-A1- 102018 101 215

## Beschreibung

Die Erfindung betrifft ein Verfahren zur Bestimmung einer optimalen Frequenz einer oszillierenden Bewegung eines durch eine Kraft beschleunigten Projektils einer intrakorporalen Lithotripsievorrichtung sowie eine entsprechende Lithotripsievorrichtung.

Die Lithotripsie ist eine im Stand der Technik bekannte Methode zur Zertrümmerung von Steinen, welche auch Konkremente genannt werden, in den Harnwegen, den Nieren und/oder der Blase. Die meisten Lithotripsiegeräte verwenden Ultraschall, Laser oder pneumatische Energiequellen, um solche Steine zu zertrümmern.

Ein im Stand der Technik bekannter Lithotripter enthält einen Schaft, der mit einem elektrisch gesteuerten Treiber oder einem pneumatischen Aktuator verbunden ist. Der Schaft wird in die Anatomie des Patienten an eine Stelle in der Nähe des Steins eingeführt, und durch den Schaft wird eine Wellenform geschickt, um den Stein mit dem Schaft zu zerschlagen und einen Presslufthammer- oder Bohreffekt auf den Stein zu erzeugen, wodurch der Stein in kleinere, leichter zu entfernende Elemente fragmentiert wird, welche dann mittels einer Saug-Spül-Pumpe entfernt werden können. Es handelt sich hierbei um ein Verfahren der intrakorporalen Lithotripsie.

Um die Steinzertrümmerung so effektiv wie möglich zu gestalten, ist es bekannt, zwei Systeme zu kombinieren, zum Beispiel die Methoden mit Ultraschall und pneumatisch erzeugten mechanischem Stoß.

Es ist ebenfalls bekannt, dass das Ultraschall- und das Pneumatiksystem unabhängig oder gemeinsam betrieben werden können. Falls sie gemeinsam betrieben werden und das pneumatische angetriebene Projektil auf die Sonotrode schlägt, so kommt es zu einer Störung des Ultraschallgenerators. Wenn das Projektil auf die Sonotrode schlägt, wirkt der Schlag auch auf ein die Ultraschallwellen erzeugendes Piezoelement, sodass eine Spannung im Generator induziert wird.

Bei bestimmten Lithotripsiesystemen kann aufgrund dieser Störung die Regelung nicht mehr aufrechterhalten werden, woraufhin eine Störmeldung angezeigt wird und das System neu gestartet werden muss.

Es ist bekannt, dass beim pneumatischen System möglichst energiereiche Schläge mit einer möglichst hohen bzw. optimalen Frequenz erzielt werden sollen. Weiterhin ist bekannt, dass der Druck, die Masse und die Beschleunigungsstrecke die Energie, welche beim Auftreffen des Projektils auf die Sonotrode abgegeben wird, maßgeblich beeinflussen.

EP 1 163 883 A1 offenbart eine Vorrichtung zum Entfernen von Körpersteinen mit einem intrakorporalen Lithotripter und einem elektrisch angesteuerten Ultraschallwandler zur longitudinalen Schwingungsanregung einer Metallsonde, wobei die über den Ultraschallwandler elektrisch angesteuerte Schwingungsanregung der Metallsonde auf eine mit einem reversibel angetriebenen Schlagteil gesteuerte Ausbildung einer Stoß- beziehungsweise Druckwelle in der Metallsonde umschaltbar ist. Der Ultraschallwandler weist wenigstens eine piezokeramische Scheibe auf, welche zwischen einem Reflektor und einem Horn angeordnet ist, wobei diese Anordnung des Ultraschallwandlers mittels einer hohlen Spannschraube verspannt ist. Ein Hohlraum der Spannschraube nimmt das reversibel angetriebene Schlagteil auf und kann mit einem Druckanschluss für einen pneumatischen Antrieb des Schlagteils ausgebildet sein.

DE 10 2018 101 215 A1 offenbart eine Vorrichtung zum Zertrümmern eines Körpersteins mit einer Sonde und einer Antriebseinheit zur Auslenkung der Sonde entlang deren Längserstreckung, wobei die Antriebseinheit eine erste Antriebseinrichtung zur periodischen Auslenkung der Sonde und eine zweite Antriebseinrichtung zur impulsförmigen Auslenkung der Sonde umfasst, wobei die Antriebseinheit derart gestaltet ist, dass eine von der zweiten Antriebseinrichtung ausgehende Wirkung auf die erste Antriebseinrichtung reduziert wird. Hierbei kann die erste Antriebseinrichtung über ein Schwingteil auf die Sonde einwirken, wobei das Schwingteil in einer senkrecht zur Längsachse verlaufenden Ebene umlaufend von aneinander anliegenden Piezoelementen umgeben ist, und/oder die zweite Antriebseinrichtung wirkt über einen Prellkörper auf die Sonde ein.

EP 1 163 882 A1 betrifft eine Vorrichtung zum Entfernen von Körpersteinen mit einer hohlen Metallsonde und einem elektrisch angesteuerten Ultraschallwandler zur longitudinalen Schwingungsanregung der hohlen Metallsonde, wobei der Ultraschallwandler mit wenigstens einer piezokeramischen Scheibe gebildet ist, welche innerhalb eines umgebenden Gehäuses zwischen einem Reflektor und einem die Hohlsonde tragenden Horn verspannt ist, wobei das Gehäuse eine mit der Hohlsonde axial fluchtende Führungshülse für ein reversibel angetriebenes Schlagteil aufnimmt, welche zur Ausübung einer Stoßkraft gegen einen Massekörper an dem über den Reflektor des Ultraschallwandlers axial nach rückwärts in die Führungshülse vorstehenden proximalen Ende einer Stoßsonde angeordnet ist, die zu ihrem distalen Ende hin durch eine axiale Durchgangsbohrung des Ultraschallwandlers hindurchgeführt und in dem Hohlraum der Hohlsonde aufgenommen ist.

Eine Aufgabe der vorliegenden Erfindung liegt darin, den Stand der Technik zu verbessern. Es ist insbesondere eine Aufgabe der Erfindung, ein störungsfrei funktionierendes Lithotripter-Ultraschall-Pneumatik-Kombinationssystem anzugeben. Es ist beispielsweise eine weitere Aufgabe der Erfindung, ein solches Kombinationssystem anzugeben, welches eine Zertrümmerungskraft eines in der Pneumatik verwendeten Projektils maximiert. Es ist insbesondere eine weitere Aufgabe der Erfindung, ein solches Kombinationssystem anzugeben, welches eine Frequenz einer wiederholten Beschleunigung eines verwendeten Projektils maximiert.

Diese Aufgabe wird durch ein Verfahren zur Bestimmung einer optimalen Frequenz einer oszillierenden Bewegung eines durch eine Kraft beschleunigten Projektils mit den Merkmalen des unabhängigen Patentanspruchs 1 gelöst. Vorteilhafte Weiterbildungen der Erfindung sind in den Unteransprüchen angegeben. Die vorliegende erfinderische Vorrichtung wird im unabhängigen Patentanspruch 9 definiert. Chirurgische oder therapeutische Verfahren werden nicht beansprucht.

### Folgendes Begriffliche sei vorab erläutert:

Unter dem vorliegenden Verfahren zur Bestimmung einer optimalen Frequenz einer oszillierenden Bewegung eines durch eine Kraft beschleunigten Projektils einer intrakorporalen Lithotripsievorrichtung, insbesondere Lithotripsievorrichtung, kann unter anderem auch ein Verfahren zum Testen, Kalibrieren, Justieren oder Optimieren der Funktionstüchtigkeit der Lithotripsievorrichtung verstanden werden. Insbesondere wird das Verfahren nicht während eines chirurgischen Eingriffs durchgeführt. Das Verfahren wird z.B. werksseitig oder vor oder entsprechend nach einem chirurgischen Eingriff durchgeführt.

Unter dem Begriff einer optimalen Frequenz der oszillierenden Bewegung des durch eine Kraft beschleunigten Projektils bei der intrakorporalen, insbesondere pneumatischen, Lithotripsie kann vorliegend eine maximale Frequenz verstanden werden. Auch wenn die Frequenz "lediglich" optimiert wird, wird vorliegend von einer optimalen Frequenz gesprochen.

Bei der vorliegenden Lithotripsie wird ein innerhalb eines abgeschlossenen Raumes laufendes Projektil von einem ersten Ende des Raumes zu einem zweiten Ende des Raumes beschleunigt, so dass das Projektil am zweiten Ende abgebremst wird. Das rasche Abbremsen des Projektils setzt eine Schlagenergie frei, die über das Projektil nach außen auf das Horn und die daran befestigte Sonotrode übertragen wird. Nach dem Abbremsen des Projektils wird das Projektil bevorzugt vom zweiten Ende zum ersten Ende zurück transportiert, worauf hin das Projektil abermals in die andere Richtung beschleunigt werden kann. Die sich wiederholende Bewegung des Projektils vom ersten Ende zum zweiten Ende und wieder zurück wird vorliegend als oszillierenden Bewegung bezeichnet.

Ausschlaggebend für die Übertragung der Schlagenergie ist ein unmittelbarer Kontakt der Spitze der Sonotrode mit dem zu zertrümmernden Konkrement.

Das Piezoelement wird bevorzugt in Resonanz mit dem Horn und der Sonotrode zum Erzeugen von Ultraschallwellen betrieben.

Die Lithotripsievorrichtung ist eine Vorrichtung zur Durchführung von Lithotripsie, insbesondere ein Handgerät mit Endoskop.

Die Ultraschallfrequenz des Piezoelements liegt bevorzugt bei 27 kHz. Das Ultraschallsignal wird bevorzugt mit einem Signalgenerator erzeugt.

Die Beschleunigungsstrecke ist vorliegend ein abgetrennter Raumbereich, in welchem das Projektil von einem Ende zum anderen Ende beschleunigt werden kann. Das Projektil kann innerhalb dieses Raumbereichs frei und bevorzugt mit wenig Reibung vor und zurück gleiten.

Die Beschleunigungsstrecke verläuft bevorzugt teilweise im Horn. Das Ende der Beschleunigungsstrecke ist bevorzugt am zweiten Anschlag mit der Sonotrode gekoppelt.

Das Gegenlager ist bevorzugt ein Reflektor für Ultraschallwellen. Das Horn wird verwendet, um die vom Piezoelement erzeugten Ultraschallwellen zur als Wellenleiter verwendete Sonotrode weiterzuleiten. Gleichzeitig dienen Gegenlager und Horn jedoch als mechanische Halterung für die Beschleunigungsstrecke, welche im Inneren des Reflektors und des Horns angeordnet ist.

Das Gegenlager und oder das Horn haben insbesondere eine hohlzylindrische Form.

Wenn das beschleunigte Projektil an dem ersten oder zweiten Anschlag abgebremst wird, so entsteht eine Erschütterung, welche durch die mechanische Kopplung ebenfalls das Piezoelement erschüttert. Aufgrund des piezoelektrischen Effektes bewirkt diese Erschütterung eine an den Enden des Piezoelements induzierte Spannung, welche mithilfe einer elektronischen Schaltung erfasst werden kann. Bei einem Auftreffen des Projektils wird im Piezoelement eine hohe Spannung erzeugt, welche die Resonanzfrequenz des Ultraschallgenerators stört. Die dort vorhandene Elektronik muss nachregeln, um wieder in Resonanz zu kommen. Dieses Nachregeln kann als Signal genutzt werden, um die Position des Projektils zu bestimmen.

Ein Kerngedanke der Erfindung besteht darin, Informationen, welche aus diesem elektrischen Signal ermittelt werden können, zum Beispiel der Zeitpunkt oder die Zeitpunkte des Auftretens der induzierten Spannung, zu verwenden, um das zeitliche Betätigen eines das Projektil beschleunigenden Mediums so zu regeln, dass eine vom Projektil auf einen zu zertrümmerndes Konkrement übertragene Kraft maximal ist und oder die Frequenz der Oszillation des Projektils im Inneren der Beschleunigungsstrecke maximiert und/oder optimiert wird.

Ein weiterer Kerngedanke der Erfindung besteht insbesondere darin, dass das Piezoelement, welches zur Erzeugung der Ultraschallwellen verwendet wird, ebenfalls als Sensor zu benutzen, um die Bewegung des Projektils im Inneren der Beschleunigungsstrecke zu optimieren.

Das Projektil kann mithilfe von Druckluft, mithilfe einer elektromagnetisch aufgeprägten Kraft oder mithilfe einer mechanischen Vorrichtung beschleunigt werden. Das die Kraft erzeugende Medium kann Druckluft, ein elektromagnetisches Feld oder eine mechanische Vorrichtung sein. Der Begriff Medium wird vorliegend im Sinne von "einem Mittel, welches eine Kraft von einer ersten Sache auf eine zweite Sache überträgt" verwendet. Unter dem Begriff des "die Kraft erzeugenden Mediums" kann vorliegend jede Vorrichtung, jeder Stoff oder jedes physikalische Feld bzw. Kraft im obigen Sinne verstanden werden. Eine Vorrichtung zum Beschleunigen des Projektils kann zum Beispiel eine Railgun sein. Alternativ oder zusätzlich können zum Beispiel mechanische Vorrichtungen zum Beschleunigen des Projektils verwendet werden.

Das Piezoelement kann mit einer Ultraschallfrequenz angeregt werden. Hierzu wird das Piezoelement bevorzugt an einem Signalgenerator angeschlossen, welcher eine Ultraschallfrequenz erzeugt.

Die Beschleunigungsstrecke kann durch ein Rohrstück realisiert werden, wobei ein erstes Ende des Rohrstücks den ersten Anschlag und ein zweites Ende des Rohrstücks den zweiten Anschlag aufweist. Das Rohrstück ist bevorzugt hohlzylinderförmig.

Ein erstes Ventil kann verwendet werden, um Druckluft in das Rohrstück einzuleiten, so dass das Projektil vom ersten Anschlag zum zweiten Anschlag beschleunigt wird. Insbesondere ist eine Druckluftquelle mithilfe eines Rückschlagventils an dem ersten Anschlag, d. h. an dem ersten Ende des Rohrstücks, angeschlossen. Weiterhin kann die vom Projektil verdrängte Luft in einer Speicherkammer, welche separat von der Beschleunigungsstrecke und/oder dem Rohrstück ist, zwischengespeichert. Diese Zwischenspeicherung ist bevorzugt kurzzeitig. Nach einem Schließen des ersten Ventils kann die zwischengespeicherte Druckluft zum Beschleunigen des Projektils vom zweiten Anschlag zum ersten Anschlag verwendet werden.

Für die Druckluft kann ein Druck zwischen 0,5 bar und 5 bar verwendet werden.

Insbesondere wird ein zweites Ventil, welches zwischen dem Rohrstück und/oder der Beschleunigungsstrecke und der Speicherkammer angeordnet ist, automatisch geöffnet, nachdem das erste Ventil geschlossen wurde, um das Projektil vom zweiten Anschlag zum ersten Anschlag zu beschleunigen.

Das erste Ventil schließt insbesondere, nachdem das Projektil den zweiten Anschlag getroffen hat.

Das elektrische Signal des Piezoelements kann ein Stromsignal, welches mithilfe einer Spule messbar ist, sein. Auf diese Art und Weise kann das elektrische Signal effektiv und mit geringen Verlusten aus dem Schaltkreis, welchen das Piezoelement aufweist, erfasst werden. Hierbei kann das Prinzip einer Strommesszange verwendet werden.

Gemäß dem erfindungsgemäßen Verfahren kann das am Piezoelement gemessene Stromsignals ferner frequenzgefiltert werden, um den Frequenzbereich, welcher um die Frequenz liegt, bei welcher das Piezoelement betrieben wird, von der Schaltung fernzuhalten, in welcher das gemessene Stromsignal weiterverarbeitet wird. Ein solches Filter könnte zum Beispiel ein RC-, RL- oder ein RLC-Glied sein.

Das frequenzgefilterte Stromsignal wird insbesondere gleichgerichtet, um ein analoges Signal zu erhalten.

Hierzu kann ein Gleichrichter oder eine Art Gleichrichter zum Einsatz kommen. Beispielsweise wird eine Prellschaltung verwendet.

Es kann ferner mindestens ein Schwellenwert des gleichgerichteten frequenzgefilterten Stromsignals ermittelt werden, welcher einem Auftreffen des Projektils am ersten oder zweiten Anschlag entspricht. Hierdurch kann in Abhängigkeit der Stromstärke, welche das Projektil beim Aufschlag auf das Piezoelement erzeugt, entschieden werden, ob es am ersten proximalen Anschlag oder dem zweiten distalen Anschlag aufgeschlagen ist. Aus dem Signal kann bevorzugt ferner der Zeitpunkt ermittelt werden, zu dem der entsprechende Anschlag erfolgt. Unter der Ortsangabe "distal" wird vorliegend eine Stelle auf einem medizinischen Gerät verstanden, welche vom Benutzer oder Operateur entfernt liegt. Unter der Ortsangabe "proximal" wird vorliegend eine Stelle auf einem medizinischen Gerät verstanden, welche nahe an einem Benutzer oder Operateur liegt.

Zur Auswertung des gleichgerichteten frequenzgefilterten Stromsignals sowie zur Auswertung der ermittelten Schwellenwerte kann ein Mikrokontroller verwendet werden. Der Mikrocontroller kann hierbei Plausibilitätstests durchführen, um falsche Messungen oder Messfehler zu minimieren oder entsprechend auszuschließen.
Das Verfahren kann mithilfe einer Regelung so durchgeführt werden, dass derjenige Spannungsausschlag, welcher von der Erschütterung bzw. des Aufpralls des Projektils am ersten Anschlag herrührt, auf einen vorgegebenen Wert geregelt wird. Der vorgegebene Wert kann der kleinste von Null unterscheidbare Wert sein, welcher mit der vorhandenen Elektronik aufgelöst werden kann. Die Regelung kann zum Beispiel eine Zweipunktregelung sein, bei der der vorgegebene Wert der Sollwert ist und der Istwert sich um den Sollwert herum bewegt. Die Regelung bewirkt insbesondere je nach Istwert ein früheres oder späteres Betätigen eines zweiten Ventils, welches in einer Speicherkammer zwischengespeicherten Druckluft freisetzen kann, so dass das Projektil vom zweiten Anschlag zum ersten Anschlag beschleunigt wird.

In einem weiteren Aspekt wird die Erfindung gelöst durch eine Lithotripsievorrichtung gemäß Anspruch 9. Die Lithotripsievorrichtung eignet sich insbesondere zur Durchführung des oben beschriebenen Verfahrens.

Die Lithotripsievorrichtung weist ein zwischen einem proximal angeordneten Gegenlager und einem distal angeordneten Horn angeordnetes Piezoelement auf, wobei das Piezoelement mechanisch an das Gegenlager und das Horn gekoppelt ist. Hierbei ist im Inneren des Gegenlagers und des Horns eine hohlzylinderförmige Beschleunigungsstrecke angeordnet, welche an einem proximalen Ende des Gegenlagers einen ersten Anschlag und an einem distalen Ende des Horns einen zweiten Anschlag aufweist.

Das proximale Ende der Beschleunigungsstrecke weist insbesondere eine mithilfe eines ersten Ventils angeschlossen Druckluftquelle auf.

Im Inneren der Beschleunigungsstrecke ist insbesondere ein Projektil angeordnet, welches ausgeführt und eingerichtet ist, mithilfe von Druckluft der Druckluftquelle vom ersten Anschlag zum zweiten Anschlag beschleunigt zu werden und mithilfe von vom Projektil verdrängten und in einer Speicherkammer zwischengespeicherten Druckluft vom zweiten Anschlag zum ersten Anschlag beschleunigt zu werden. Zwischen der Speicherkammer und der Beschleunigungsstrecke kann ein zweites Ventil angeordnet sein.

Eine als Wellenleiter ausgeführte Sonotrode ist insbesondere an einem distalen Ende des Horns angeordnet.

Hierbei ist ein proximales Ende der Sonotrode mit dem zweiten Anschlag der Beschleunigungsstrecke mechanisch gekoppelt.

Die Lithotripsievorrichtung ist hierbei insbesondere so ausgeführt und eingerichtet, dass ein elektrisches Signal des Piezoelements, welches von einer Erschütterung an dem ersten und/oder zweiten Anschlag durch das Projektil herrührt, erfassbar ist und zur Regelung der Druckluft der Druckluftquelle verwendet wird.

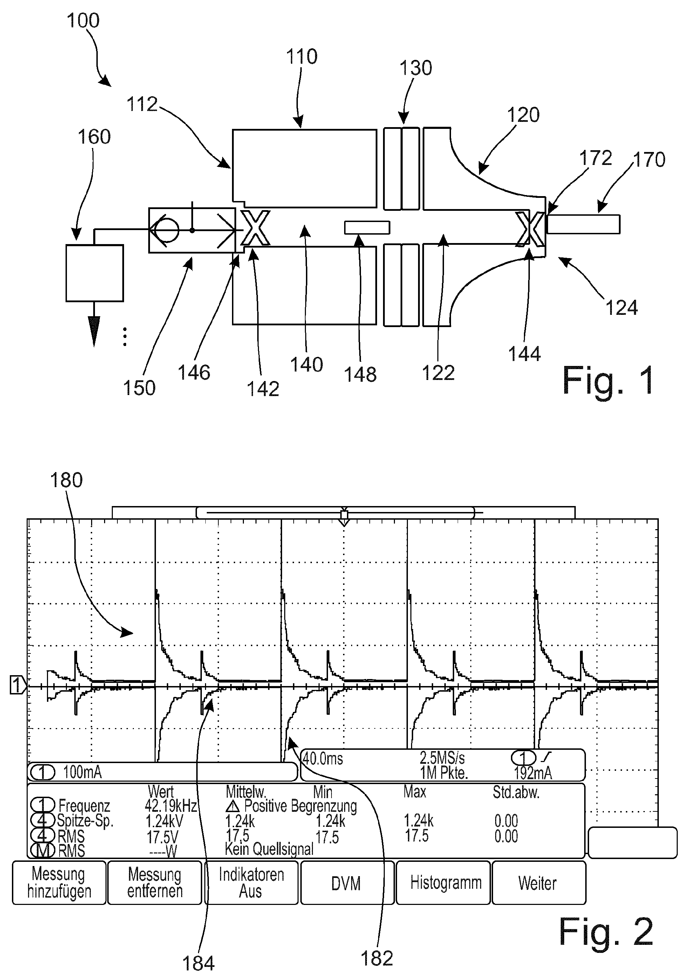

Im Weiteren wird die Erfindung anhand eines Ausführungsbeispiels näher erläutert. Es zeigen
- Figur 1: eine schematische Darstellung einer Lithotripsievorrichtung gemäß einer Ausführungsform der Erfindung und
- Figur 2: eine Messung eines an einem Piezoelement anliegenden elektrischen Stromsignales gegenüber der Zeit während der Durchführung eines Verfahrens gemäß einer Ausführungsform der Erfindung.

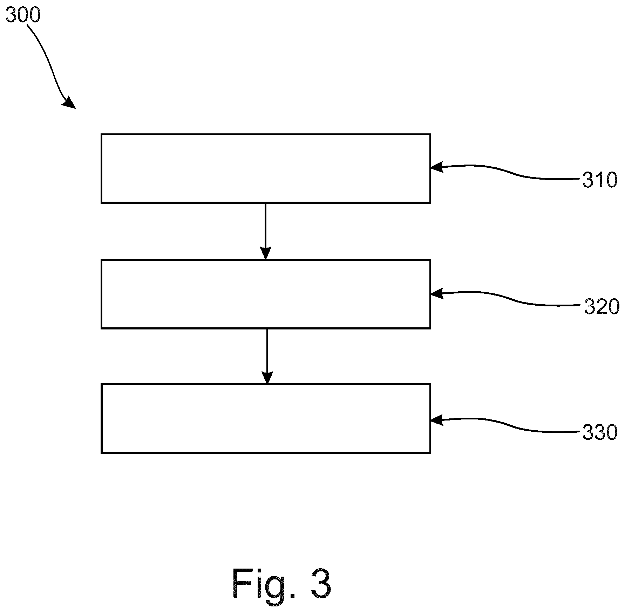

- Figur 3: zeigt ein Ablaufdiagramm eines Verfahrens gemäß Anspruch 1.

Eine Lithotripsievorrichtung 100 dient der Durchführung eines Verfahrens zum Bestimmen einer optimalen Frequenz einer oszillierenden Bewegung eines durch Druckluft angeregten Projektils. Die Lithotripsievorrichtung 100 kann zum Beispiel ein Handgerät mit einer am distalen Ende des Handgeräts angebrachten Sonotrode sein, wobei die Sonotrode einen flexiblen Wellenleiterschaft aufweist.

Die Lithotripsievorrichtung 100 weist ein zwischen einem proximal angeordneten Gegenlager 110 und einem distal angeordneten Horn 120 angeordnetes Piezoelement 130 auf. Hierbei ist das Piezoelement 130 mechanisch an das Gegenlager 110 und das Horn 120 gekoppelt. Das Piezoelement 130 wird mithilfe eines nicht dargestellten Signalgenerators mit einer Ultraschallfrequenz von ca. 27 kHz beaufschlagt.

Das Gegenlager 110 und das Piezoelement 130 haben jeweils eine hohlzylindrische Form. Das Horn 120 hat eine rotationssymmetrische Form mit einer zylinderförmigen Aushöhlung entlang einer zentralen Längsachse. Ein proximales Ende des Horns 120 weist denselben Außendurchmesser wie das Piezoelement 130 auf. Ausgehend vom proximalen Ende des Horns 120 bleibt der Außendurchmesser des Horns zunächst für eine vorgegebene Strecke konstant und fällt danach asymptotisch auf einen Wert des Durchmessers ab, welcher etwas größer als der Durchmesser der zylinderförmigen Aushöhlung im Inneren des Horns 120 ist.

Das Gegenlager 110 weist vorliegend die Funktion eines Reflektors für die vom Piezoelement 130 erzeugten Ultraschallwellen auf. Durch die Formgebung des Horns 120 und/oder des Gegenlagers 110 kann erreicht werden, dass die erzeugten Quer- und Rotationsschwingungen ebenso wie die erzeugten Longitudinalschwingungen optimal an ein distales Ende der Sonotrode 170 geleitet werden. Hierbei ist es vorteilhaft, dass die Sonotrode 170 und das Horn 120 aus Werkstoffen im Wesentlichen gleicher akustischer Impedanz bestehen.

Im Inneren 122 des Gegenlagers 110 und des Horns 120 befindet sich ein hohlzylinderförmige Rohrstück 140, dessen erstes Ende an einem proximalen Ende 112 des Gegenlagers 110 einen ersten Anschlag 142 und dessen zweites Ende an einem distalen Ende 124 des Horns 120 einen zweiten Anschlag 144 aufweist.

Ein proximales Ende 146 des Rohrstücks 140 weist einen mithilfe eines ersten Ventils 150 angeschlossene Druckluftquelle 160 auf. Das erste Ventil 150 weist ein Rückschlagventil auf.

Im Inneren 122 des Rohrstücks 140 ist ein längliches Projektil 148 angeordnet, welches mithilfe von Druckluft der Druckluftquelle 160 vom ersten Anschlag 142 zum zweiten Anschlag 144 beschleunigt werden kann. Das Projektil 148 kann im Rohrstück 140 nach vorne und zurück frei gleiten. Das Projektil 148 kann mithilfe von vom Projektil 148 verdrängten und in einer nicht abgebildeten Speicherkammer zwischengespeicherten Druckluft vom zweiten Anschlag 144 zum ersten Anschlag 142 beschleunigt werden.

Das Projektil 148 weist einen zylindrischen Körper aus äußerst hartem Stahl auf, welcher schwach magnetisch ist. Am proximalen Ende 146 des Rohrstücks 140 ist ein nicht abgebildeter Haltemagnet angeordnet, welcher das Projektil 148 dorthin ziehen und im Ruhezustand dort festhalten kann.

Eine als Wellenleiter ausgeführte Sonotrode 170 ist an einem distalen Ende 124 des Horns 120 angeordnet. Hierbei ist ein proximales Ende 172 der Sonotrode 170 mit dem zweiten Anschlag 144 mechanisch gekoppelt, sodass ein Auftreffen des Projektils 148 auf den zweiten Anschlag 144 den Impuls des Projektils 148 optimal auf die Sonotrode 170 überträgt. Ein Durchmesser der Sonotrode 170 ist kleiner als ein Durchmesser des Rohrstücks 140.

Bei der pneumatischen Lithotripsie können beide Systeme, d. h. das Ultraschallsystem mit dem Ultraschallelement 130 und das pneumatische System, bei dem das Projektil 148 mithilfe der Druckluft der Druckluftquelle 160 beschleunigt wird, verwendet werden. Dies wird Kombinationsbetrieb genannt. Alternativ kann das pneumatische System auch ohne das Ultraschallsystem betrieben werden. In letzterem Fall kann das Gesamtsystem bei ausgeschaltetem Signalgenerator kalibriert werden, d. h. es können die Grenzwerte einer Stromstärke hinterlegt werden und als Referenz im Kombinationsbetrieb dienen, bei dem eine Messung schwieriger ist, da die Ultraschallfrequenz eine Störquelle darstellt.

In beiden Fällen, d. h. im Kombinationsbetrieb oder falls lediglich das pneumatische System betrieben wird, wird mithilfe einer nicht dargestellten Strommesszange an einer Verbindungsleitung zwischen Piezoelement 130 und Signalgenerator ein Stromsignal gemessen.

Da die Ultraschallschwingungen des Piezoelements 130 eine Störquelle darstellen, wird das mit der Strommesszange gemessene Stromsignal mit einem RLC-Glied frequenzgefiltert, so dass ein kleiner Bereich um die Ultraschallfrequenz von ca. 27 kHz aus dem Stromsignal herausgefiltert wird. Die Breite der herausgefilterten Frequenz ist bevorzugt an das Störsignal angepasst.

Das frequenzgefilterte Stromsignal wird mithilfe einer Prellschaltung in ein analoges gleichgerichtetes Signal umgewandelt.

Bei dem gleichgerichteten Signal können zwei Schwellenwerte ermittelt werden, ein erster Schwellenwert entspricht einem Auftreffen des Projektils 148 am ersten Anschlag 142 und ein zweiter Schwellenwert entspricht einem Auftreffen des Projektils am zweiten Anschlag 144.

Das gleichgerichtete Signal wird von einem Mikrocontroller aufgezeichnet, welcher die gesamte Auswertung steuert und kontrolliert. Der Mikrocontroller kann Plausibilitätstests des erfassten Stromsignals durchführen, um Fehlmessungen oder Messfehler zu minimieren.

Das Verfahren zur Bestimmung einer maximalen oder entsprechend optimalen Frequenz einer oszillierenden Bewegung des durch Druckluft angeregten Projektils 148 weist gemäß einem ersten Schritt ein wiederholtes Beschleunigen des Projektils 148 mithilfe von Druckluft vom ersten proximalen Anschlag 142 des Rohrstücks 140 zu einem zweiten distalen Anschlag 144 und vom zweiten Anschlag 144 zum ersten Anschlag 140 auf.

Hierbei wird das erste Ventil 150 verwendet, um Druckluft in das Rohrstück 140 einzuleiten, so dass das Projektil 148 vom ersten Anschlag 142 zum zweiten Anschlag 144 beschleunigt wird, wobei die vom Projektil 148 verdrängte Luft in einer Speicherkammer zwischengespeichert wird, und nach einem Schließen des ersten Ventils 150 die zwischengespeicherte Druckluft zum Beschleunigen des Projektils 148 vom zweiten Anschlag 144 zum ersten Anschlag 142 verwendet wird.

Gemäß einem zweiten Schritt des Verfahrens wird das Piezoelement 130 mit einer Ultraschallfrequenz angeregt. Hierzu wird ein nicht dargestellter bei 27 kHz betriebener Signalgenerator an das Piezoelement 130 angeschlossen.

Gemäß einem dritten Schritt des Verfahrens wird ein Stromsignal des Piezoelements 130 erfasst, welches von einer Erschütterung am ersten Anschlag 142 oder zweiten Anschlag 144 durch das Projektil 148 herrührt.

Gemäß einem vierten Schritt des Verfahrens wird das erfasste Stromsignal zur Regelung der Druckluft verwendet.

Das Stromsignal 180 weist mehrere exponentiell abfallende mit einer Sinus- oder Kosinusfunktion modellierte Abschnitte auf, welche zeitlich voneinander getrennt sind. Hierbei rührt ein erster Abschnitt 182 des Stromsignals 180 von einer Erschütterung des Projektils 148 an dem zweiten Anschlag 144 und ein zweiter Abschnitt 184 des Stromsignals 180 von einer Erschütterung an dem ersten Anschlag 142.

### Bezugszeichenliste

- 100: Lithotripsievorrichtung
- 110: Gegenlager
- 112: proximales Ende des Gegenlagers
- 120: Horn
- 122: Inneres des Gegenlagers und des Horns
- 124: distales Ende des Horns
- 130: Piezoelement
- 140: Rohrstück
- 142: erster Anschlag der Bescheinigungstrecke
- 144: zweiter Anschlag der Bescheinigungstrecke
- 146: proximales Ende der Beschleunigungsstrecke
- 148: Projektil
- 150: erstes Ventil
- 160: Druckluftquelle
- 170: Sonotrode
- 172: proximales Ende der Sonotrode
- 180: Stromsignal
- 182: erster Abschnitt des Stromsignals
- 184: zweiter Abschnitt des Stromsignals
- 300: Verfahren
- 310: Verfahrensschritt
- 320: Verfahrensschritt
- 330: Verfahrensschritt

## Patentansprüche

1. Verfahren (300) zur Bestimmung einer optimalen Frequenz einer oszillierenden Bewegung eines durch eine Kraft beschleunigten Projektils (148) einer intrakorporalen Lithotripsievorrichtung (100) vor oder nach einem chirurgischen Eingriff,
- wobei ein wiederholtes Beschleunigen (310) des Projektils (148) von einem ersten proximalen Anschlag (142) einer Beschleunigungsstrecke zu einem zweiten distalen Anschlag (144) und vom zweiten Anschlag (144) zum ersten Anschlag (140) durchgeführt wird, wobei ein Piezoelement (130) zwischen einem proximal angeordneten Gegenlager (110) und einem distal angeordneten Horn (120) angeordnet ist und mechanisch an das Gegenlager (110) und das Horn (120) gekoppelt ist, und das Horn (120) eine distal angeordnete Sonotrode (170) aufweist, wobei die Beschleunigungsstrecke im Inneren (122) des Gegenlagers (110) und des Horns (120) angeordnet ist, und der erste Anschlag (142) an einem distalen Ende (112) des Gegenlagers (110) und der zweite Anschlag (144) an einem distalen Ende (124) des Horns (120) angeordnet ist, mit folgenden Schritten:
- Erfassen (320) eines elektrischen Signals des Piezoelements (130), welches von einer Erschütterung an dem ersten Anschlag (142) und / oder zweiten Anschlag (144) durch das Projektil (148) herrührt; und
- Verwenden (330) des erfassten elektrischen Signals zur Regelung eines die Kraft erzeugenden Mediums, welches zur Beschleunigung des Projektils (148) vom ersten Anschlag (142) der Beschleunigungsstrecke zu dem zweiten Anschlag (144) und vom zweiten Anschlag (144) zum ersten Anschlag (142) verwendet wird.

2. Verfahren (300) nach Anspruch 1, **dadurch gekennzeichnet, dass** das Projektil (148) mithilfe von Druckluft, mithilfe einer elektromagnetisch aufgeprägten Kraft oder mithilfe einer mechanischen Vorrichtung beschleunigt wird und/oder dass das Medium Druckluft, ein elektromagnetisches Feld oder eine mechanische Vorrichtung ist.

3. Verfahren (300) nach Anspruch 1 oder 2,
ferner aufweisend:
- Anregen des Piezoelements (130) mit einer Ultraschallfrequenz.

4. Verfahren (300) nach Anspruch 1, 2 oder 3, **dadurch gekennzeichnet, dass** die Beschleunigungsstrecke durch ein Rohrstück (140) realisiert wird, wobei ein erstes Ende des Rohrstücks (140) den ersten Anschlag (142) und ein zweites Ende des Rohrstücks (140) den zweiten Anschlag (144) aufweisen.

5. Verfahren (300) nach Anspruch 4, **dadurch gekennzeichnet, dass** ein erstes Ventil (150) verwendet wird, um Druckluft in das Rohrstück (140) einzuleiten, so dass das Projektil (148) vom ersten Anschlag (142) zum zweiten Anschlag (144) beschleunigt wird, wobei die vom Projektil (148) verdrängte Luft in einer Speicherkammer zwischengespeichert wird, und nach einem Schließen des ersten Ventils (150) die zwischengespeicherte Druckluft zum Beschleunigen des Projektils (148) vom zweiten Anschlag (144) zum ersten Anschlag (142) verwendet wird.

6. Verfahren (300) nach einem der vorangegangenen Ansprüche, **dadurch gekennzeichnet, dass** das elektrische Signal des Piezoelements (130) ein Stromsignal ist, welches mithilfe einer Spule gemessen wird.

7. Verfahren (300) nach dem vorangegangenen Anspruch,
ferner aufweisend:
- Frequenzfiltern des am Piezoelement (130) gemessenen Stromsignals und
- Gleichrichten des frequenzgefilterten Stromsignals.

8. Verfahren (300) nach dem vorangegangenen Anspruch,
ferner aufweisend:
- Ermitteln mindestens eines Schwellenwertes des gleichgerichteten frequenzgefilterten Stromsignals, welcher einem Auftreffen des Projektils (148) am ersten Anschlag (142) oder zweiten Anschlag (144) entspricht.

9. Lithotripsievorrichtung (100), aufweisend:
- ein zwischen einem proximal angeordneten Gegenlager (110) und einem distal angeordneten Horn (120) angeordnetes Piezoelement (130), wobei das Piezoelement (130) mechanisch an das Gegenlager (110) und das Horn (120) gekoppelt ist und im Inneren (122) des Gegenlagers (110) und des Horns (120) eine hohlzylinderförmige Beschleunigungsstrecke angeordnet ist, welche an einem proximalen Ende (112) des Gegenlagers (110) einen ersten Anschlag (142) und an einem distalen Ende (124) des Horns (120) einen zweiten Anschlag (144) aufweist, wobei ein proximales Ende (146) der Beschleunigungsstrecke eine mithilfe eines ersten Ventils (150) angeschlossene Druckluftquelle (160) aufweist oder die Lithotripsievorrichtung (100) eine Vorrichtung zum Erzeugen eines elektromagnetisches Feldes zur Ausübung einer elektromagnetisch auf ein Projektil (148) aufgeprägten Kraft aufweist; und im Inneren (122) der Beschleunigungsstrecke das Projektil (148) angeordnet ist, welches ausgeführt und eingerichtet ist, mithilfe von Druckluft der Druckluftquelle (160) oder der elektromagnetisch aufgeprägten Kraft vom ersten Anschlag (142) zum zweiten Anschlag (144) beschleunigt zu werden,
- eine als Wellenleiter ausgeführte Sonotrode (170), welche an einem distalen Ende (124) des Horns (120) angeordnet ist, wobei ein proximales Ende (172) der Sonotrode (170) mit dem zweiten Anschlag (144) mechanisch gekoppelt ist,
**dadurch gekennzeichnet, dass** das Projektil (148) ausgeführt und eingerichtet ist, mithilfe von vom Projektil (148) verdrängten und in einer Speicherkammer zwischengespeicherten Druckluft oder der elektromagnetisch aufgeprägten Kraft vom zweiten Anschlag (144) zum ersten Anschlag (142) beschleunigt zu werden und die Lithotripsievorrichtung (100) so ausgeführt und eingerichtet ist, dass ein elektrisches Signal des Piezoelements (130), welches von einer Erschütterung an dem ersten Anschlag (142) und/oder zweiten Anschlag (144) durch das Projektil (148) herrührt, erfassbar ist und zur Regelung der Druckluft der Druckluftquelle (160) oder zur Regelung der elektromagnetisch aufgeprägten Kraft verwendet wird.

## Claims

1. A method (300) for determining an optimal frequency of an oscillating movement of a force-accelerated projectile (148) of an intracorporeal lithotripsy device (100) before or after a surgical procedure,
- wherein the projectile (148) is repeatedly accelerated (310) from a first proximal stop (142) of an acceleration path to a second distal stop (144) and from the second stop (144) to the first stop (140), wherein a piezo element (130) is arranged between a proximally arranged counter bearing (110) and a distally arranged horn (120) and is mechanically coupled to the counter bearing (110) and the horn (120), and the horn (120) has a distally arranged sonotrode (170), wherein the acceleration path is arranged in the interior (122) of the counter bearing (110) and the horn (120), and the first stop (142) is arranged at a distal end (112) of the counter bearing (110) and the second stop (144) is arranged at a distal end (124) of the horn (120), having the following steps:
- detecting (320) an electrical signal of the piezo element (130), caused by a shock at the first stop (142) and/or second stop (144) as a result of the projectile (148); and
- using (330) the detected electrical signal to regulate a force-generating medium which is used to accelerate the projectile (148) from the first stop (142) of the acceleration path to the second stop (144) and from the second stop (144) to the first stop (142).

2. The method (300) according to claim 1, **characterised in that** the projectile (148) is accelerated by means of compressed air, by means of an electromagnetically imparted force or by means of a mechanical device and/or **in that** the medium is compressed air, an electromagnetic field or a mechanical device.

3. The method (300) according to claim 1 or 2,
further comprising:
- exciting the piezo element (130) with an ultrasonic frequency.

4. The method (300) according to claim 1, 2 or 3, **characterised in that** the acceleration path is implemented by a pipe section (140), wherein a first end of the pipe section (140) has the first stop (142) and a second end of the pipe section (140) has the second stop (144).

5. The method (300) according to claim 4, **characterised in that** a first valve (150) is used to introduce compressed air into the pipe section (140) such that the projectile (148) is accelerated from the first stop (142) to the second stop (144), wherein the air displaced by the projectile (148) is temporarily stored in a storage chamber, and after closing the first valve (150), the temporarily stored compressed air is used to accelerate the projectile (148) from the second stop (144) to the first stop (142).

6. The method (300) according to one of the preceding claims, **characterised in that** the electrical signal of the piezo element (130) is a current signal which is measured by means of a coil.

7. The method (300) according to the preceding claim,
further comprising:
- frequency-filtering the current signal measured at the piezo element (130) and
- rectifying the frequency-filtered current signal.

8. The method (300) according to the preceding claim,
further comprising:
- determining at least one threshold value of the rectified, frequency-filtered current signal, which corresponds to the projectile (148) impacting the first stop (142) or second stop (144).

9. A lithotripsy apparatus (100), comprising:
- a piezo element (130) arranged between a proximally arranged counter bearing (110) and a distally arranged horn (120), wherein the piezo element (130) is mechanically coupled to the counter bearing (110) and the horn (120) and in the interior (122) of the counter bearing (110) and the horn (120) is arranged a hollow-cylindrical acceleration path, which has a first stop (142) at a proximal end (112) of the counter bearing (110) and a second stop (144) at a distal end (124) of the horn (120), wherein a proximal end (146) of the acceleration path has a compressed air source (160) connected by means of a first valve (150) or the lithotripsy device (100) has a device for generating an electromagnetic field for exerting a force imparted electromagnetically on a projectile (148); and in the interior (122) of the acceleration path is arranged the projectile (148), which is designed and configured to be accelerated from the first stop (142) to the second stop (144) by means of compressed air from the compressed air source (160) or the electromagnetically imparted force,
- a sonotrode (170) which is designed as a waveguide and is arranged at a distal end (124) of the horn (120), wherein a proximal end (172) of the sonotrode (170) is mechanically coupled to the second stop (144), **characterised in that** the projectile (148) is designed and configured to be accelerated from the second stop (144) to the first stop (142) by means of compressed air displaced by the projectile (148) and temporarily stored in a storage chamber or the electromagnetically imparted force, and the lithotripsy device (100) is designed and configured such that an electrical signal of the piezo element (130), caused by a shock at the first stop (142) and/or second stop (144) as a result of the projectile (148), can be detected and is used to regulate the compressed air of the compressed air source (160) or to regulate the electromagnetically imparted force.

## Revendications

1. Procédé (300) de détermination d'une fréquence optimale d'un mouvement oscillant d'un projectile (148) accéléré par une force d'un dispositif de lithotripsie intracorporelle (100) avant ou après une intervention chirurgicale,
- dans lequel une accélération répétée (310) du projectile (148) est effectuée depuis une première butée (142) proximale d'un trajet d'accélération jusqu'à une seconde butée (144) distale et depuis la seconde butée (144) jusqu'à la première butée (140), dans lequel un élément piézoélectrique (130) est agencé entre un contre-appui (110) agencé de manière proximale et un cornet (120) agencé de manière distale et est accouplé mécaniquement au contre-appui (110) et au cornet (120), et le cornet (120) présente une sonotrode (170) agencée de manière distale, dans lequel le trajet d'accélération est agencé dans l'intérieur (122) du contre-appui (110) et du cornet (120), et la première butée (142) est agencée au niveau d'une extrémité distale (112) du contre-appui (110) et la seconde butée (144) est agencée au niveau d'une extrémité distale (124) du cornet (120), comportant les étapes suivantes :
- la détection (320) d'un signal électrique de l'élément piézoélectrique (130) lequel résulte d'une vibration au niveau de la première butée (142) et/ou de la seconde butée (144) par le projectile (148) ; et
- l'utilisation (330) du signal électrique détecté pour commander un milieu générant la force, lequel est utilisé pour l'accélération du projectile (148) depuis la première butée (142) du trajet d'accélération jusqu'à la seconde butée (144) et depuis la seconde butée (144) jusqu'à la première butée (142).

2. Procédé (300) selon la revendication 1, **caractérisé en ce que** le projectile (148) est accéléré à l'aide d'air comprimé, à l'aide d'une force appliquée de manière électromagnétique ou à l'aide d'un dispositif mécanique et/ou **en ce que** le milieu est de l'air comprimé, un champ électromagnétique ou un dispositif mécanique.

3. Procédé (300) selon la revendication 1 ou 2,
présentant en outre:
- l'excitation de l'élément piézoélectrique (130) à une fréquence ultrasonore.

4. Procédé (300) selon la revendication 1, 2 ou 3, **caractérisé en ce que** le trajet d'accélération est réalisé par un tronçon de tube (140), dans lequel une première extrémité du tronçon de tube (140) présente la première butée (142) et une seconde extrémité du tronçon de tube (140) présente la seconde butée (144).

5. Procédé (300) selon la revendication 4, **caractérisé en ce qu'**une première soupape (150) est utilisée pour introduire de l'air comprimé dans le tronçon de tube (140), de sorte que le projectile (148) est accéléré depuis la première butée (142) jusqu'à la seconde butée (144), dans lequel l'air déplacé par le projectile (148) est stocké temporairement dans une chambre de stockage, et après une fermeture de la première soupape (150), l'air comprimé stocké temporairement est utilisé pour accélérer le projectile (148) depuis la seconde butée (144) jusqu'à la première butée (142).

6. Procédé (300) selon l'une des revendications précédentes, **caractérisé en ce que** le signal électrique de l'élément piézoélectrique (130) est un signal de courant, lequel est mesuré à l'aide d'une bobine.

7. Procédé (300) selon la revendication précédente,
présentant en outre :
- le filtrage en fréquence du signal de courant mesuré sur l'élément piézoélectrique (130) et
- le redressement du signal de courant filtré en fréquence.

8. Procédé (300) selon la revendication précédente,
présentant en outre :
- la détermination d'au moins une valeur seuil du signal de courant redressé et filtré en fréquence, laquelle correspond à un impact du projectile (148) sur la première butée (142) ou la seconde butée (144).

9. Dispositif de lithotripsie (100), présentant :
- un élément piézoélectrique (130) agencé entre un contre-appui (110) agencé de manière proximale et un cornet (120) agencé de manière distale, dans lequel l'élément piézoélectrique (130) est accouplé mécaniquement au contre-appui (110) et au cornet (120) et un trajet d'accélération en forme de cylindre creux est agencé à l'intérieur (122) du contre-appui (110) et du cornet (120), lequel présente une première butée (142) au niveau d'une extrémité proximale (112) du contre-appui (110) et une seconde butée (144) au niveau d'une extrémité distale (124) du cornet (120), dans lequel une extrémité proximale (146) du trajet d'accélération présente une source d'air comprimé (160) raccordée à l'aide d'une première soupape (150) ou le dispositif de lithotripsie (100) présente un dispositif de génération d'un champ électromagnétique pour exercer une force appliquée de manière électromagnétique sur un projectile (148) ; et à l'intérieur (122) du trajet d'accélération est agencé le projectile (148) lequel est réalisé et conçu pour être accéléré depuis la première butée (142) jusqu'à la seconde butée (144) à l'aide d'air comprimé provenant de la source d'air comprimé (160) ou de la force appliquée de manière électromagnétique,
- une sonotrode (170) réalisée comme un guide d'ondes, laquelle est agencée au niveau d'une extrémité distale (124) du cornet (120), dans lequel une extrémité proximale (172) de la sonotrode (170) est accouplée mécaniquement à la seconde butée (144),
**caractérisé en ce que** le projectile (148) est réalisé et conçu pour être accéléré depuis la seconde butée (144) jusqu'à la première butée (142) à l'aide d'air comprimé déplacé par le projectile (148) et stocké temporairement dans une chambre de stockage ou de la force appliquée de manière électromagnétique et le dispositif de lithotripsie (100) est réalisé et conçu de telle sorte, qu'un signal électrique de l'élément piézoélectrique (130), lequel résulte d'une vibration au niveau de la première butée (142) et/ou de la seconde butée (144) par le projectile (148), peut être détecté et est utilisé pour la régulation de l'air comprimé de la source d'air comprimé (160) ou pour la régulation de la force appliquée de manière électromagnétique.
